# EUROPÄISCHE PATENTSCHRIFT

(11) **EP 2 285 414 B1**
(45) Veröffentlichungstag und Bekanntmachung des Hinweises auf die Patenterteilung: **10.04.2013**
(21) Anmeldenummer: 09757186.3
(22) Anmeldetag: 22.05.2009
(51) Int. Cl.: A61K 47/48, C07K 14/765, A61L 31/08, A61K 9/16

(54) **VOLLSYNTHETISCHES ALBUMIN-ANALOGON**
FULLY SYNTHETIC ALBUMIN ANALOGUE
ANALOGUE TOTALEMENT SYNTHÉTIQUE DE L'ALBUMINE

(30) Priorität: 30.05.2008 DE 102008027133
(43) Veröffentlichungstag der Anmeldung: 23.02.2011
(73) Patentinhaber: LS medcap GmbH, 72379 Hechingen (DE)
(72) Erfinder: LEHMANN, Hans-Dieter, 72406 Bisingen-Zimmern (DE)
(74) Vertreter: Witte, Weller & Partner
(86) Internationale Anmeldenummer: PCT/EP2009/003651
(87) Internationale Veröffentlichungsnummer: WO 2009/146803

(56) Entgegenhaltungen:
- WO-A1-03/094991
- WO-A1-2007/019994
- FR-A1- 2 665 902
- US-A1- 2002 019 037
- US-A1- 2004 142 011
- US-A1- 2006 015 057
- WINBLADE N D; ET AL: "STERICALLY BLOCKING ADHESION OF CELLS TO BIOLOGICAL SURFACES WITH A SURFACE-ACTIVE COPOLYMER CONTAINING POLY(ETHYLENE GLYCOL) AND PHENYLBORONIC ACID" JOURNAL OF BIOMEDICAL MATERIALS RESEARCH, WILEY, NEW YORK, NY, US, Bd. 59, Nr. 4, 15. März 2002 (2002-03-15), Seiten 618-631, XP008065440 ISSN: 0021-9304

## Beschreibung

Die vorliegende Erfindung betrifft eine Verbindung, die in Beschichtungen für mit Blut in Kontakt kommende Oberflächen einsetzbar ist, um die Hämatokompatibilität der Oberfläche zu verbessern, sowie eine derartige Beschichtung und eine medizinische Vorrichtung mit zumindest einer derartigen Oberfläche.

Derartige Verbindungen, Beschichtungszusammensetzungen sowie Verfahren zur Beschichtung von Oberflächen medizinischer Vorrichtungen sind im Stand der Technik vielfältig bekannt.

Bei vielen medizinischen Behandlungen werden derartige medizinische Vorrichtungen verwendet, die Kunststoffoberflächen aufweisen, die über längere oder kürzere Zeitdauer mit dem Blut eines Patienten in Kontakt gelangen. Bei diesen Vorrichtungen handelt es sich bspw. um Einmalgeräte für eine Herz-Lungen-Maschine, Oxygenatoren, Dialysatoren, Ultrafilter, Katheter, künstliche Organe wie Herz oder Niere, Gasaustauschmembranen oder vaskuläre Prothesen, wobei diese Aufzählung nicht als abschließend zu verstehen ist.

Bei all diesen medizinischen Geräten aktiviert der dabei erfolgende Kontakt des Blutes mit hydrophoben Polymeroberflächen verschiedene Abwehrmechanismen im Blut. Dies gilt insbesondere bei der Durchleitung von Blut durch Dialysatoren oder Ultrafilter, die hydrophile Membranen aufweisen, aber auch bei der Durchleitung von Blut durch Oxygenatoren mit hydrophoben Membranen und mit großflächigen Entschäumern, wobei hier weit höhere Fließgeschwindigkeiten des Blutes vorliegen.

Hier sind nicht nur das strömungsbedingte Trauma, sondern vor allem auch die Eigenschaften der Kunststoffoberflächen selbst für unerwünschte Reaktionen im Blut verantwortlich.

Dabei kann es zur Anhaftung von Blutbestandteilen an Kunststoffoberflächen und zur Koagulation kommen, was zum einen die Gefahr von Funktionsstörungen der Geräte und zum anderen die Gefahr von Thrombosen erhöht. Darüber hinaus kommt es überwiegend zu einer Aktivierung von im Blut befindlichen Bestandteilen des Immunsystems, insbesondere des Komplementsystems. Eine solche Aktivierung der Immunantwort ist aber insbesondere hinsichtlich des beeinträchtigten Gesundheitszustandes der Patienten von Nachteil und daher zu verhindern.

Daher ist es wünschenswert, für mit Blut in Kontakt kommende medizinische Geräte und Zubehör Kunststoffoberflächen verfügbar zu haben, die eine gute Blutverträglichkeit aufweisen.

Unter guter Blutverträglichkeit, also Hämatokompatibilität, wird allgemein die Eigenschaft einer Oberfläche oder Substanz verstanden, dass sie bei Kontakt mit Blut weder die Blutgerinnung noch die Abwehrmechanismen des Körpers gegen die Fremdoberfläche oder Substanz aktiviert.

Um dies zu erreichen, ist es bekannt, die mit Blut in Kontakt kommenden Kunststoffoberflächen in einer Weise zu beschichten, dass diese eine verbesserte Hämatokompatibilität aufweisen.

Dabei ist zu beachten, dass bspw. Dialysatorgehäuse, Oxygenatoren und entsprechendes Zubehör vorzugsweise aus klarem Polycarbonat gefertigt sind, dessen Durchsichtigkeit und Erscheinungsbild durch derartige hydrophilisierende Beschichtungen nicht beeinträchtigt werden darf. Weiter ist zu beachten, dass derartige Beschichtungen vor der Sterilisation der entsprechenden medizinischen Geräte durchgeführt werden, um beim Einsatz industriell fertig vorbereiteter Geräte ein Bluttrauma zu reduzieren. Dies ist vor dem Hintergrund wichtig, dass blutverträgliche und sterile medizinische Vorrichtungen benötigt werden, die vorrätig gehalten werden können und jederzeit und vor allem im Notfall einsatzbereit sind.

In diesem Zusammenhang ist es bspw. bekannt, die Blutgerinnung durch eine hochdosierte Gabe von Heparin zu hemmen oder aber Heparin an solchen Oberflächen zu binden, die mit Blut in Kontakt kommen.

Weiterhin ist es bekannt, eine gewisse Hämatokompatibilität von Kunststoffoberflächen durch das Vorspülen mit Patientenblut oder durch Beschichtung mit Blutbestandteilen menschlicher, tierischer oder biotechnologischer Herkunft zu erzielen.

Hierbei ist es vor allem das im Blut in großen Mengen vorhandene Protein Albumin, welches rasch von den hydrophoben Oberflächen adsorbiert wird und dann an diesen Oberflächen denaturiert, wodurch die Oberflächen hydrophilisiert werden.

Ferner ist es bekannt, durch die Beschichtung von Kunststoffoberflächen mit synthetischen Tensiden die Hämatokompatibilität in oder an medizinischen Geräten und/oder Zubehör zu verbessern.

Die insoweit beschriebenen Verfahren weisen jedoch alle bestimmte Nachteile auf.

Die Hemmung der Blutgerinnung mittels Heparin ist insbesondere in der Notfallmedizin und besonders bei Patienten, die evtl. mechanische oder chemische Traumata aufweisen, kontraindiziert, da es hierdurch zu verstärkten Blutungen kommen kann.

Bei dem Vorspülen mit Patientenblut ist insbesondere von Nachteil, dass die Beschichtung erst unmittelbar vor dem Einsatz der so zu beschichtenden Vorrichtungen erfolgen kann. Somit würde bei Notfallbehandlungen wichtige Zeit verloren gehen, was zur erheblichen gesundheitlichen Beeinträchtigung des Patienten führen könnte. Weiterhin birgt diese Behandlung den Nachteil, dass sie nur zeitlich eng begrenzt eingesetzt werden kann. Da das sich beim Vorspülen der Vorrichtung mit Patientenblut an die lipophilen Oberflächen anlagernde Albumin leicht durch andere lipophile Substanzen von den Oberflächen verdrängt wird, kommt es hierbei zu einer stetigen Verschlechterung der Hämatokompatibilität.

Auch kann das Denaturieren von Albumin an den lipophilen Oberflächen zur vermehrten Freisetzung von an das Albumin gebundenen lipophilen Substanzen wie Medikamenten oder Toxinen führen, wodurch die Wirkung eingesetzter Medikamente schwerer einschätzbar wird und sich toxische Effekte einstellen können. Weiterhin ist mit diesem Verfahren keine Kontrolle der Beschichtungsqualität möglich.

Bei der Beschichtung mit Blutbestandteilen menschlichen oder tierischen Ursprungs ist insbesondere die Infektionsgefahr ein Faktor, der die behördliche Zulassung derartiger Beschichtungen massiv erschwert. Darüber hinaus kann es bei derartigen Beschichtungen zu Unverträglichkeitsreaktionen kommen.

Die Beschichtung mit biotechnologisch gewonnenen Bestandteilen ist dagegen insbesondere wegen der im Zusammenhang mit der Gewinnung und Aufreinigung dieser Substanzen anfallenden Kosten von Nachteil.

Um diesen Problemen sowie den Zulassungsproblemen bei Albuminbeschichtungen zu begegnen, besteht deshalb ein Bedarf an physiologisch unbedenklichen, neuartigen Beschichtungsmaterialien für die Hydrophilisierung von Polymeroberflächen, wobei hier auch das Fehlen von Viren und anderen biologischen Risikofaktoren vorausgesetzt werden kann.

Für die hämatokompatible Beschichtung von Kunststoffoberflächen werden daher vermehrt synthetische Substanzen wie Polymere oder Tenside eingesetzt.

In diesem Zusammenhang beschreibt die US 6,670,199 verschiedene Beschichtungen, die als Grundgerüst das Tensid Pluronic™ enthalten, das mit verschiedenen Biomolekülen konjugiert sein kann.

In der WO 2007/01994 wurde gezeigt, dass diese Beschichtungen zwar antithrombogene Eigenschaften aufweisen, aber gleichzeitig auch zu einer starken Verschlechterung der Komplement-Aktivierung führen. Die bekannte Beschichtung weist damit keine gute Blutverträglichkeit auf. Sie hat weiterhin den Nachteil, dass die Gleichmäßigkeit der Beschichtung nur schwer zu kontrollieren ist.

Eine weitere Forderung an Beschichtungsverfahren besteht darin, dass es im Betrieb der mit entsprechend beschichteten Oberflächen versehenen medizinischen Geräte nicht zu einem Auswaschen von Beschichtungsmaterial aus den beschichteten Oberflächen kommen darf. Ein solches Auswaschen könnte nämlich nach der Rückführung des Blutes in den Blutkreislauf des Patienten toxische Wirkungen, wie z.B. das Auslösen entzündlicher Reaktionen hervorrufen.

Es ist bekannt, dass synthetische Substanzen im Blut fast durchweg als fremd empfunden werden und Abwehrmechanismen auslösen. Die Anbindung von Polyethylenoxidgruppen (PEO) an derartige Fremdmoleküle wirkt maskierend und vermag somit immunologische Abwehrreaktionen zu unterdrücken. In diesem Zusammenhang offenbart die WO 2007/019994 A1 die Verwendung bestimmter Estertenside in hämatokompatiblen Beschichtungen von lipophilen Oberflächen. Diese Estertenside weisen bis zu sechs langkettige Fettsäurereste auf, an die Polyethylenoxidketten gebunden sind. Wegen dieser gebundenen PEO-Reste sind die Tenside in Wasser löslich.

Die immunologische Maskierung durch PEO-Gruppen wird auch bei der Herstellung von künstlichem Blut aus Hämoglobin tierischen Ursprungs genutzt, wie es bspw. aus der WO 2002/000230 A1 bekannt ist. Hierzu werden durch Anbinden von Polyethylenoxidketten körperfremde Substanzen maskiert, wodurch die Auslösung der Immunantwort verringert werden kann.

Aus der oben erwähnten WO 2007/019994 A1 ist es bekannt, dass aufgrund der Kombination eines großen lipophilen Molekülanteils, der über Van-der-Waals-Kräfte eine relativ starke Anheftung an lipophile Oberflächen erlaubt, mit angebundenen PEO-Ketten, die die Substanz gegenüber den Komponenten der Immunantwort maskieren, mit einem der beschriebenen Estertenside, welches als Cremophor™ vertrieben wird, eine gegenüber Pluronic™ verringerte Komplement-Aktivierung erzielt werden kann.

Vergleichs-Versuche im Hause der Anmelderin haben jedoch gezeigt, dass es auch im Blut, das mit mit Cremophor™ beschichteten Oberflächen in Kontakt gelangt, zu Veränderungen gegenüber identischem, nicht mit Oberflächen in Kontakt gebrachtem humanem Blut kommt, die auf eine starke Aktivierung von Komponenten des Immunsystems hinweisen.

Vor diesem Hintergrund liegt der vorliegenden Erfindung daher die Aufgabe zugrunde, eine neue Klasse von Verbindungen bereitzustellen, die in Beschichtungen für mit Blut in Kontakt kommenden Oberflächen verwendet werden können, und die gegenüber bekannten Verbindungen verbesserte hämatokompatible Eigenschaften aufweisen.

Erfindungsgemäß wird diese Aufgabe gelöst durch die Bereitstellung eines vollsynthetischen Albumin-Analogons mit mindestens einer dreidimensionalen Grundstruktur, die mindestens zwei gebundene Gelenkbereiche in geometrisch zueinander definierter Weise aufweist, an denen mindestens ein Rest kovalent gebunden ist.

Die der Erfindung zugrunde liegende Aufgabe wird auf diese Weise vollkommen gelöst.

Der Erfinder der vorliegenden Anmeldung hat nämlich erkannt, dass durch die Verwendung der neuen Albumin-Analoga in Beschichtungen die Hämatokompatibilität von mit Blut in Kontakt kommenden Kunststoffoberflächen gegenüber den im Stand der Technik bekannten Beschichtungssubstanzen und -verfahren deutlich verbessert wird.

Vor diesem Hintergrund betrifft die vorliegende Erfindung auch eine hämatokompatible Beschichtung für mit Blut in Kontakt kommende Oberflächen, die mindestens ein neues, vollsynthetisches Albumin-Analogon enthält.

Ferner betrifft die vorliegende Erfindung eine medizinische Vorrichtung mit zumindest einer derartigen Oberfläche, sowie die Verwendung des neuen Albumin-Analogons in einer Beschichtung für mit Blut in Kontakt kommende Polymeroberflächen.

Bei dem neuen Albumin-Analogon ist von Vorteil, dass sich durch das erfindungsgemäß verwendete, dreidimensionale Grundgerüst ein lipophiler Molekülanteil darstellen lässt, der eine höhere räumliche Dichte aufweist als bekannte Substanzen, bei denen lineare Aliphate als Linker verwendet werden.

Dies führt zum einen zu einer stärkeren Van-der-Waals-Interaktion der neuen Albumin-Analoga mit lipophilen Oberflächen und damit zu einer besseren Anhaftung, es begünstigt aber aufgrund der geringeren Raumforderung des lipophilen Molekülanteils auch die Löslichkeit in wässrigem Medium.

Die gute Löslichkeit in wässrigem Medium ist deshalb vorteilhaft, weil es beim Beschichten von Kunststoffoberflächen mit organische Lösungsmittel aufweisenden Beschichtungsmitteln zu Spannungsrisskorrosion kommen kann.

Die Gelenkbereiche definieren die räumliche Anordnung der kovalent daran gebunden Reste und bieten so den Vorteil, dass es bei Anhaftung der neuen Verbindung an eine lipophile Oberfläche zu einem organisierten Verteilungsmuster der hydrophilen Molekülanteile auf der Oberfläche kommt.

Damit lässt sich mit dem neuen Albumin-Analogon eine homogene Besetzung der Oberfläche mit Hämatokompatibilität vermittelnden Molekülanteilen bewirken, wie es so bei bekannten Substanzen mit räumlich undefinierter Grundstruktur nicht möglich oder bekannt ist.

All dies führt bei Verwendung des neuen Albumin-Analogons zu einer wesentlichen Verbesserung der Beschichtungsqualität bei gleichzeitig verringerter Menge der einzusetzenden Beschichtungssubstanzen.

Im Hause der Anmelderin wurden drei verschiedene Ausführungsbeispiele des neuen Albumin-Analogons im Chandler-Loop-Test hinsichtlich ihrer hämatokompatiblen Eigenschaften getestet und mit den Eigenschaften von Cremophor®EL verglichen. Hierbei zeigten sich die neuen Albumin-Analoga insbesondere hinsichtlich der nach der Exposition im getesteten Blut nachgewiesenen Platelet-Zahl und Thromboglobulin-Konzentration, die ein Maß für die Aktivierung des Immunsystems darstellt, als dem bekannten Cremophor®EL überlegen.

Weiterhin hat der Erfinder festgestellt, dass das neue Albumin-Analogon nierengängig ist, so dass es im Gegensatz zu Humanalbumin über die Niere aus Patientenblut eliminiert werden kann.

Vor diesem Hintergrund betrifft die vorliegende Erfindung auch die Verwendung des neuen Albumin-Analogons zur intrakorporalen Detoxifikation.

Die neue Verbindung wird als Albumin-Analogon bezeichnet, weil es wie Humanalbumin in Blut und in wässrigen Lösungen in kolloidaler Form vorliegt. Es hat hydrophile und hydrophobe Bereiche. Die hydrophilen Bereiche bedingen dabei die Löslichkeit in Wasser, während die hydrophoben Anteile im Lösungszustand im Inneren des Molekül-Ellipsoids vorliegen und Taschen bilden, in denen lipophile Substanzen transportiert werden können, z.B. hydrophobe Medikamente oder, im Fall von Hepatitis, Bilirubin.

Die neuen Albumin-Analoga weisen nämlich sehr hohe Affinitäten zu hydrophoben Substanzen auf und sind auch als Toxin-belasteter Komplex noch nierengängig. Dadurch wird die Möglichkeit geboten, durch Zuführen einer erfindungsgemäßen Substanz, im Blut befindliche toxische lipophile Substanzen in einem Komplex zu binden und über die Nieren auszuscheiden.

Bei dem neuen Albumin-Analogon ist es dabei bevorzugt, wenn die dreidimensionale Grundstruktur ausgewählt ist aus der Gruppe umfassend einfache Aromate, kondensierte Aromate, einfache Heteroaromate, kondensierte Heteroaromate und Hydrierungsprodukte der zuvor genannten Stoffklassen.

Hier ist von Vorteil, dass derartige mono- bzw. polycyclische Verbindungen eine definierte dreidimensionale Struktur aufweisen, die in dem erfindungsgemäßen Albumin-Analogon eine vorteilhafte Organisiertheit der Grundstrukturen und Reste sowie eine hohe räumliche Dichte lipophiler Molekülanteile bewirkt.

Im Falle der kondensierten Aromaten, einfachen Heteroaromaten und/oder kondensierten Heteroaromaten liegt eine im Wesentlichen planare Geometrie vor, so dass im Zusammenwirken der Grundstrukturen mit den lipophilen Anteilen der daran gebundenen Reste eine ideale Kontaktfläche für die Bildung von Van-der-Waals-Interaktionen mit lipophilen Oberflächen gebildet wird.

Im Falle der Hydrierungsprodukte von Aromaten oder Heteroaromaten ergibt sich eine vorteilhafte Wirkung einerseits aus der Möglichkeit, Gelenkbereiche in definierten Positionen entweder axial oder äquatorial zu den Grundstrukturen anzubinden, wodurch sich bspw. mit einer differenziellen Besetzung axialer oder äquatorialer Gelenkbereiche mit lipophilen bzw. hydrophilen Resten räumlich organisierte Molekülanteile verschiedener Funktion darstellen lassen. Andererseits ergibt sich eine vorteilhafte Wirkung aus der relativen Flexibilität einer hydrierten Ringstruktur, die, bei gleichzeitigem weitgehenden Erhalt einer definierten Ausrichtung, eine effiziente Anheftung und Van-der-Waals-Interaktion auch mit stark strukturierten Polymeroberflächen ermöglicht.

In einer Weiterbildung ist bevorzugt, wenn der mindestens eine Rest innere Bereiche aufweist, die an den Gelenkbereichen gebunden sind, und äußere Bereiche aufweist, die an den inneren Bereichen gebunden sind.

Hierbei ist von Vorteil, dass die inneren und äußeren Bereiche, die unterschiedliche Eigenschaften hinsichtlich bspw. ihrer Hydrophilie aufweisen, durch Um- bzw. Zusammenlagerung Ausformungen, z.B. einen Kern und einen Mantel, bilden können. Diese Ausformungen wiederum können hinsichtlich bspw. ihrer Hydrophilie in energetisch bevorzugter Weise mit verschiedenen Medien oder Oberflächen interagieren.

Dabei ist bevorzugt, wenn die inneren Bereiche lipophile Bereiche und die äußeren Bereiche hydrophile Bereiche aufweisen.

Bei in Lösung in wässrigem Medium befindlichen Verbindungen ist es hier von Vorteil, dass die innen angeordneten lipophilen Bereiche durch die außen angeordneten hydrophilen Bereiche vom umgebenden Medium abgeschirmt werden, was zu einer erhöhten Löslichkeit führt.

Bei an einer lipophilen Oberfläche assoziierten Verbindungen ist hingegen von Vorteil, dass die lipophilen Bereiche der Grundstruktur und der Reste nahe zusammenliegen, bzw. einen durchgehenden lipophilen Molekülanteil bilden, was eine effiziente Van-der-Waals-Interaktion mit der lipophilen Oberfläche ermöglicht.

Dabei ist bevorzugt, wenn die lipophilen Bereiche durch Alkylketten gebildet werden, die starke Van-der-Waals-Interaktion mit lipophilen Oberflächen ausbilden.

Weiterhin ist bevorzugt, wenn hydrophile Bereiche durch Polyethylen-Oxid-Ketten (PEO), gebildet werden, die hydrophile Eigenschaften aufweisen, welche eine gute Wasserlöslichkeit der neuen Albumin-Analoga und die Bildung einer hydrophilen Kontaktfläche mit antithrombogener und hämatokompatibler Wirkung ermöglichen.

PEO-Ketten sind darüber hinaus, wie in der o.g. WO 2002/000230 A1 beschrieben, geeignet, körperfremde Substanzen gegenüber dem Immunsystem zu maskieren und so die Auslösung der Immunantwort zu vermindern.

Weiterhin dienen die Alkylketten der zusätzlichen Vergrößerung des lipophilen Molekülanteils, um die Bindung der neuen Albumin-Analoga an lipophile Oberflächen zu verstärken. Die PEO-Ketten vermitteln hingegen die Löslichkeit in wässrigem Medium, z.B. in einer Beschichtungslösung, um eine Beschichtung durch Spülen auf eine Oberfläche aufzubringen.

Weiterhin ist bevorzugt, wenn ein Kettenabschluss an Resten mit organischen Resten abgesättigt ist.

Da endständige Hydroxyl-Gruppen zur Aktivierung des Komplement-Systems beitragen ist hierbei von Vorteil, dass ein durch Absättigung mit einem organischen Rest, z.B. durch eine Methoxylgruppe oder durch Acetylierung erzeugtes Kettenende im Blutkontakt die Komplement-Antwort nicht oder nur geringfügig auslöst.

In einer Weiterbildung ist bevorzugt, wenn Reste miteinander durch mindestens eine Brücke verbunden sind.

Hierbei ist von Vorteil, dass zusätzlich zur räumlichen Organisation der Reste über die Grundstrukturen durch Brücken ein nochmals erhöhter räumlicher Organisierungsgrad der Reste zueinander erzeugt wird.

Hierbei ist dann darauf zu achten, dass durch die eingeführte räumliche Definiertheit der Reste zueinander weder die Löslichkeit in wässrigen Medien noch die Anheftung an lipophile Oberflächen noch der Umlagerungsprozess beim Anhaften in Lösung befindlicher Moleküle an eine lipophile Oberfläche signifikant verringert bzw. behindert werden.

In einer Ausführungsform der neuen Albumin-Analoga ist bevorzugt, wenn es zumindest zwei Grundstrukturen aufweist, die über zumindest eine Verbrückung miteinander verbunden sind.

Hierbei ist es von Vorteil, dass sich über die Verbindung mehrerer Grundstrukturen miteinander der Grad der räumlichen bzw. flächigen Organisiertheit weiter erhöhen lässt. Dies führt zu einer weiteren Steigerung der Beschichtungsqualität.

Weiterhin kann, analog zu Proteinstrukturen wie bspw. Albumin, durch das Zusammenlagern der lipophilen Molekülanteile im Inneren des Moleküls die Löslichkeit in wässrigem Medium weiter verbessert und gleichzeitig der Bildung von Aggregaten aus mehreren Molekülen entgegengewirkt werden.

Außerdem lässt sich über die Anzahl miteinander durch eine Verbrückung verbundener Grundstrukturen auch die Anzahl der an die Grundstrukturen gebundenen Reste im Gesamtmolekül variieren.

Weiterhin ist bevorzugt, wenn die Verbrückung eine Alkylbrücke der Formel (CH₂)ₙ mit einer Länge von n = 2 bis 18 aufweist.

Dabei ist es vorteilhaft, dass sich mittels einer Alkylbrücke zwischen zwei Grundstrukturen ein größerer zusammenhängender lipophiler Molekülanteil darstellen lässt, was wiederum die Ausbildung von Van-der-Waals-Interaktionen erhöht.

Weiterhin ist bevorzugt, wenn die Verbrückung mindestens eine Verzweigung aufweist, an der ein Rest gebunden ist.

Hierbei ist von Vorteil, dass bspw. über das Einführen einer lipophilen Verzweigung an der Verbrückung der lipophile Molekülanteil weiter vergrößert werden kann.

Weiterhin können bei einer ein- oder mehrfach verzweigten Verbrückung hydrophile Reste wie bspw. PEO-Ketten eingeführt werden, die durch die verbesserte Abschirmung des lipophilen Molekülanteils gegenüber dem wässrigen Medium bzw. Blut die Löslichkeit und Hämatokompatibilität des Moleküls erhöhen.

Gemäß einer Weiterbildung der neuen Albumin-Analoga ist bevorzugt, wenn an einigen Gelenkbereichen, Resten und/oder Verbrückungen hydrophile Funktionen, bspw. ionische Gruppen wie etwa Carboxylat-, Phosphat- oder Sulfonat-Anionen, eingeführt sind.

Hierbei ist von Vorteil, dass die Einführung ionischer Gruppen die Kontrolle der Qualität einer Beschichtung mit den neuen Albumin-Analoga erleichtert. So können anionische Gruppen eingeführt werden, die für Kontrollzwecke mit kationischen Farbstoffen angefärbt und anschließend visuell oder automatisch detektiert werden. Zusammenfassend ist bei den neuen Albumin-Analoga, die in wässriger Lösung oder Emulsion zur Verwendung kommen, von Vorteil, dass sie einen hohen lipophilen Anteil besitzen können und dennoch für das Beschichten mittels wässriger Medien geeignet sind. Im wässrigen Medium sind die lipophilen Bereiche durch die hydrophilen Bereiche vollständig abgeschirmt. An lipophilen Kunststoffoberflächen entfaltet sich der lipophile Anteil der neuen Albumin-Analoga in geeigneter Weise, so dass er über Van-der-Waals-Interaktionen an den zu beschichteten Oberflächen gut anhaftet. Diese Entfaltung ist durch geeignete Gelenkbereiche bedingt, die die räumliche Umordnung ermöglichen.

Vor dem Hintergrund der obigen Ausführungen kann eine hämatokompatible Beschichtung für mit Blut in Kontakt kommende Oberflächen mindestens ein vollsynthetisches Albumin-Analogon aufweisen.

Diese hämatokompatible Beschichtung kann - wie bereits erwähnt - auf mit Blut in Kontakt kommende, lipophile Oberflächen, bspw. von medizinischen Vorrichtungen, aufgebracht, diesen gute hämatokompatible und antithrombogene Eigenschaften vermitteln. Dadurch wird die Aktivierung des Komplementsystems im Blut vermindert und einer Adsorption und Koagulation von Erythrozyten oder Leukozyten an den Oberflächen entgegengewirkt.

Dabei ist bevorzugt, wenn die hämatokompatible Beschichtung eine oder mehrere hämatokompatible detektierbare Substanzen aufweist.

Hierbei ist von Vorteil, dass im Zuge der Qualitätssicherung die Beschichtungsqualität jedes einzelnen hergestellten medizinischen Geräts, das eine erfindungsgemäße Beschichtung aufweist, überprüft werden kann, ohne dass hierzu zusätzliche Verfahren, wie z.B. das Spülen mit einer eine detektierbare Substanz enthaltenden Flüssigkeit, notwendig sind.

Gleichzeitig bleiben die weiteren vorteilhaften Eigenschaften der Beschichtung, insbesondere die Hämatokompatibilität, erhalten.

Eine medizinische Vorrichtung mit zumindest einer Kunststoffoberfläche aus Polycarbonat, Polyethylen, Polypropylen, Polymethylpenten, Polyurethan, Polyester, Silikon, Hart- oder Weich-Polyvinylchlorid, Copolymer wie z.B. Acrylnitril-Butadien-Styrol-Copolymer, Ethylen-Propylen-(Dien)-Copolymer etc. kann erfindungsgemäß mit den neuen Albumin-Analoga beschichtet werden.

Dabei ist bevorzugt, wenn die medizinische Vorrichtung Bestandteil von Einmalgeräten einer Herz-Lungen-Maschine, eines Oxygenators, eines Katheters, eines künstlichen Herzens, einer künstlichen Niere, einer Gasaustauschmembran oder einer vaskulären Prothese ist.

Weitere Vorteile ergeben sich aus der beigefügten Beschreibung und den Tabellen.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung ist in den Figuren beispielhaft dargestellt, in denen:
- Fig. 1: eine schematisierte Darstellung einer möglichen Ausführungsform der neuen Albumin-Analoga mit außen angeordneten hydrophilen Molekülanteilen zeigt;
- Fig. 2 e: ine schematisierte Darstellung einer möglichen Ausführungsform der neuen Albumin-Analoga mit außen angeordneten lipophilen Molekülanteilen zeigt;
- Fig. 3: eine schematisierte Darstellung einer möglichen Ausführungsform der neuen Albumin-Analoga mit zwei über eine Verbrückung verbundenen Grundstrukturen zeigt;
- Fig. 4: die Formel der in den konkreten Ausführungsformen der neuen Albumin-Analoga verwendeten Grundstruktur zeigt;
- Fig. 5: die Formel zweier durch eine Verbrückung verbundener Grundstrukturen in einer konkreten Ausführungsform der neuen Albumin-Analoga zeigt;
- Fig. 6: die Formel der in den konkreten Ausführungsformen der neuen Albumin-Analoga an die Grundstrukturen gebundenen Reste zeigt; und
- Fig. 7: einen stark vergrößerten schematischen Ausschnitt einer Oberfläche mit einer erfindungsgemäßen Beschichtung zeigt.

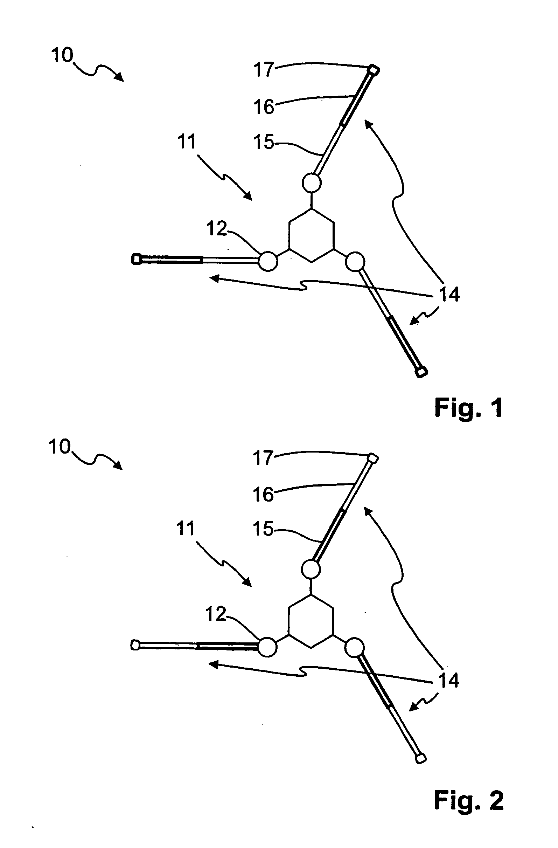

In Fig. 1 ist eine schematisierte Darstellung einer möglichen Ausführungsform der neuen Albumin-Analoga 10 dargestellt. Hierbei sind an eine dreidimensionale Grundstruktur 11 über Gelenkbereiche 12 Reste 14 kovalent gebunden. Diese Reste 14 weisen jeweils einen inneren Bereich 15, einen äußeren Bereich 16 und einen Kettenabschluss 17 auf, wobei der innere Bereich 15 lipophile Charakteristika aufweist, der äußere Bereich 16 und der Kettenabschluss 17 hingegen hydrophile Charakteristika aufweisen.

In dieser und den folgenden Figuren sind hydrophile Molekülanteile an den Resten mit dicker Linierung gekennzeichnet. Die mehrfach dargestellten Molekülbestandteile wie bspw. Gelenkbereiche 12 sind in der Zeichnung aus Gründen der Übersichtlichkeit nur beispielhaft mit Bezugszeichen versehen.

Diese Ausführungsform hat den Vorteil eines insgesamt kompakten Molekülaufbaus, wobei die Reste radialsymmetrisch und planar am Grundgerüst angeordnet sind. Es wird somit an Oberflächen ein hoher Organisiertheitsgrad erzielt. In Lösung hingegen werden durch Umlagerung der Reste eine gute Kompaktierung des lipophilen Molekülanteils und eine effiziente hydrophile Ummantelung erzielt.

Die in Fig. 2 dargestellte Ausführungsform entspricht weitgehend der Ausführungsform in Fig. 1. Jedoch weisen in diesem Fall der äußere Bereich 16 und der Kettenabschluss 17 der Reste 14 lipophile Charakteristika auf, während der innere Bereich 15 der Reste 14 hydrophil ist.

Diese Ausführungsform hat den Vorteil, dass in den neuen Albumin-Analoga 10, wenn sie an einer lipophilen Oberfläche assoziiert vorliegen, die für die Hämatokompatibilität kritischen Kettenabschlüsse 17 von der Lösung abgewandt sind.

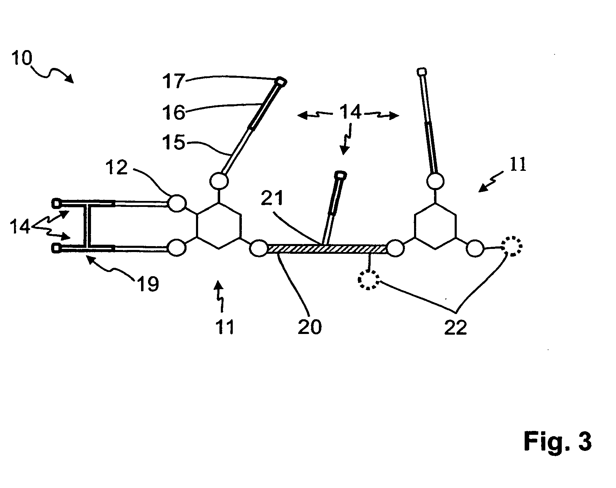

Fig. 3 zeigt eine schematisierte Darstellung einer möglichen Ausführungsform der neuen Albumin-Analoga, in der zwei Grundstrukturen 11 über eine Verbrückung 20 miteinander verbunden sind.

Wie bei den vorhergehenden Figuren 1 und 2, sind an dreidimensionalen Grundstrukturen 11 über Gelenkbereiche 12 Reste 14 kovalent gebunden. Diese Reste 14 sind in einen inneren Bereich 15 und einen äußeren Bereich 16 untergliedert. Weiterhin weisen die Reste 14 einen Kettenabschluss 17 auf.

Die Untergliederung der Reste 14 in einen inneren Bereich 15 und einen äußeren Bereich 16 hat zum Vorteil, dass ein größerer Radius der hydrophilen Abschirmung erzeugt werden kann, die lineare Abfolge lipophiler und hydrophiler Anteile aber zugleich die räumliche Organisation dieser Molekülanteile bedingt. Somit lässt sich leichter eine gleichmäßige Besetzung einer lipophilen Oberfläche mit hydrophilen Molekülanteilen in einer Beschichtung erzielen.

Hier sind auch einteilige Reste 14 möglich, die aufgrund der durch die enge Bindung der Reste 14 an die Grundstruktur 11 bedingten sterischen Einschränkung eine vergleichsweise rigide Molekülgeometrie bei eingeschränktem Radius der hydrophilen Abschirmung bewirken.

Brücken 19, die die Reste 14 miteinander verbinden leisten auch einen Beitrag zur sterischen Einschränkung der Ausrichtung der Reste 14.

Weiterhin ist zwischen den Grundstrukturen 11 eine Verbrückung 20 vorgesehen, an der weiterhin eine Verzweigung 21 mit einem Rest 14 vorgesehen ist.

An Gelenkbereichen 12 und der Verbrückung 20 sind eingeführte hydrophile Funktionen 22, bspw. ionische Gruppen wie etwa Carboxylat-, Phosphat- oder Sulfonat-Anionen, dargestellt.

Neben ihrer Hydrophilizität ermöglichen es diese Gruppen mittels kationischer Färbemittel die Beschichtungsqualität zu prüfen.

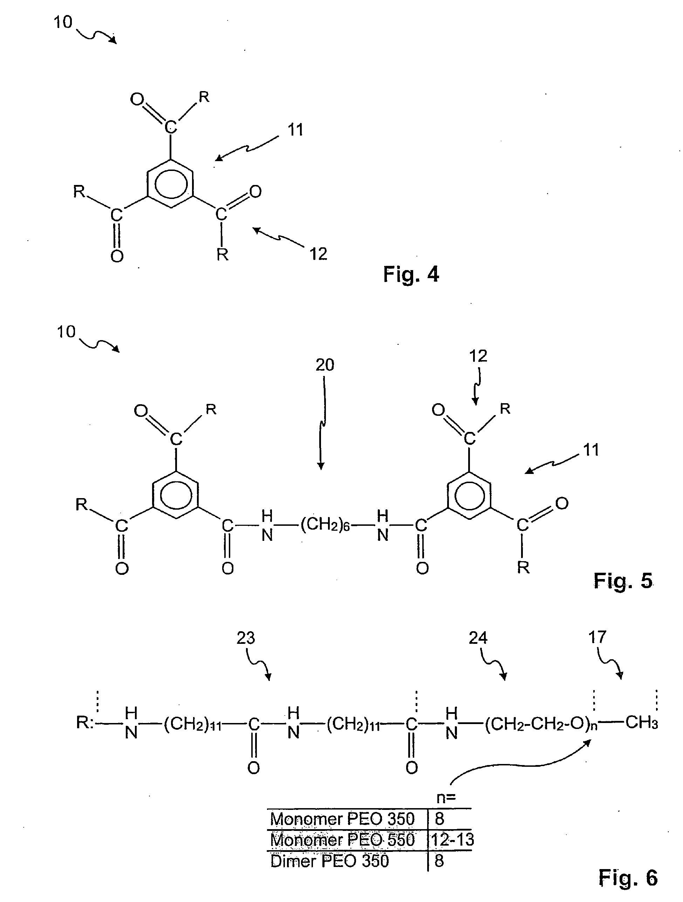

Fig. 4 zeigt eine Grundstruktur 11 der in den konkreten Ausführungsbeispielen angeführten Substanzen, welche im Folgenden als "Monomer PEO 350", "Monomer PEO 550" und "Dimer PEO 350" bezeichnet werden.

Analog zu dem in Fig. 1 dargestellten Schema sind hier an eine dreidimensionale Grundstruktur 11, die aus einer 1,3,5-Benzotricarbonsäure-Gruppe besteht, über Gelenkbereiche 12, welche durch die Säureamidbindungen gebildet werden, mit "R" bezeichnete Reste kovalent gebunden (Formel der Reste siehe Fig. 6).

Fig. 5 zeigt den Aufbau des neuen Albumin-Analogons, welches im Folgenden als "Dimer PEO 350" bezeichnet wird.

Dieses Dimer weist zwei Grundstrukturen 11 auf, die je aus einer 1,3,5-Benzotricarbonsäure-Gruppe bestehen, und die über eine Verbrückung 20 miteinander verbunden sind, welche aus einer Alkylkette der Formel (CH₂)ₙ der Länge n = 6 besteht, die jeweils über als Gelenkbereiche 12 fungierende Säureamidbindungen an die Grundstrukturen gebunden ist, wobei die Grundstrukturen 11 jeweils zwei über als Gelenkbereiche 12 fungierende Säureamidbindungen gebundene Reste "R" aufweisen.

Fig. 6 zeigt das Grundgerüst der in den konkreten Ausführungsbeispielen der neuen Albumin-Analoga gebundenen Reste "R". Diese weisen Alkylketten als lipophile Bereiche 23 und PEO-Ketten als hydrophile Bereiche 24 auf. Der Kettenabschluss 17 an den PEO-Ketten erfolgt durch eine Methoxyl-Gruppe.

Hierbei weisen die Substanzen "Monomer PEO 350" und "Dimer PEO 350" PEO-Ketten der Formel (CH₂-CH₂-O)ₙ mit der Länge n = 8 (ausgehend von der dimeren Aminoundecansäure) und mit einem Molekulargewicht von ca. 350 Dalton (Da) auf. Die Substanz "Monomer PEO 550" weist PEO-Ketten der Länge n = 12-13 (ausgehend von der dimeren Aminoundecansäure) mit einem Molekulargewicht von ca. 550 Dalton (Da) auf.

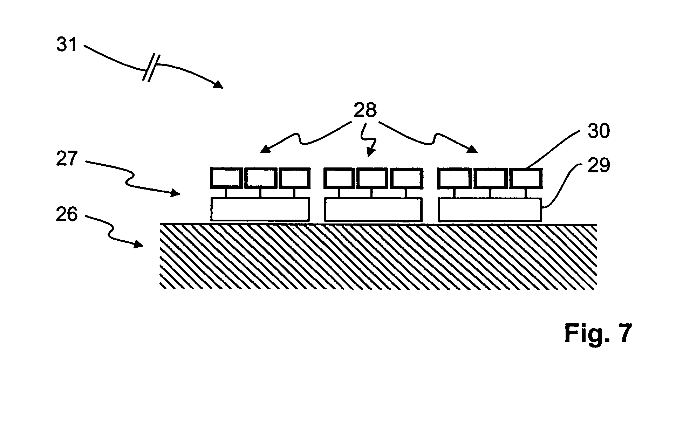

In Fig. 7 ist schematisch ein stark vergrößerter Ausschnitt einer Oberfläche 26 dargestellt, die eine Beschichtung 27 mit den neuen Albumin-Analoga 28 aufweist. Die Beschichtung 27 ist über die lipophilen Anteile 29 der neuen Albumin-Analoga 28 an die Oberfläche 26 gebunden. Über die hydrophilen Anteile 30 der neuen Albumin-Analoga 28 steht die Beschichtung mit einem physiologischen wässrigen Medium 31, bspw. Blut, in Kontakt und schirmt die Oberfläche 26 gegenüber dem Medium 31 ab.

Die drei unter Fig. 4 bis 6 aufgeführten neuen Albumin-Analoga wurden hinsichtlich ihrer Hämatokompatibilität und antithrombogenen Wirkung charakterisiert und mit dem bekannten Cremophor®EL verglichen. Dabei konnte der Erfinder nachweisen, dass die neuen Albumin-Analoga insbesondere gemessen an der nach der Exposition im getesteten Blut nachgewiesenen Platelet-Zahl und der Thromboglobulin-Konzentration, die ein Maß für die Aktivierung des Immunsystems darstellen, dem bekannten Cremophor®EL hinsichtlich ihrer Hämatokompatibilität überlegen sind. Hierzu wurden die drei unter Fig. 4 bis 6 aufgeführten neuen Albumin-Analoga einzeln zur Beschichtung einer Polycarbonat-Oberfläche verwendet und in einem Chandler-Loop-Test mit Blut in Kontakt gebracht. Im Anschluss wurde anhand mehrerer Parameter die Hämatokompatibilität und Antithrombogenizität der beschichteten Oberflächen bestimmt. Als Kontrollen wurden frisches Donorblut, unbeschichtete Polycarbonat-Oberflächen und mit dem bekannten Cremophor®EL beschichtete Polycarbonat-Oberflächen verwendet.

Zur Bestimmung der Hämatokompatibilität und Antithrombogenizität der beschichteten Oberflächen wurde ein so genannter Chandler-Loop-Test durchgeführt.

### Herstellung der Probenkörper (Loops)

Für die Herstellung der Loops wurden zunächst 7 handelsübliche Polycarbonat-Konnektoren über 3/8" Silikonschlauchstücke miteinander verbunden. Zur Beschichtung wurden 1000 ml wässrige Beschichtungslösung unter Verwendung von 1 m 3/8" Pumpschlauch aus Silikon mit einer Schlauchpumpe bei einem Fluss von zwei Litern pro Minute für 20 Minuten bei Raumtemperatur im Kreislauf umgepumpt. Nach der Beschichtung wurde die Beschichtungslösung verworfen und die beschichteten Loops ohne weitere Spülung mit steriler Pressluft ausgeblasen und dadurch getrocknet. Nach einer Endtrocknung von vier Stunden bei 40°C im Trockenschrank war der Beschichtungsprozess beendet. Die verwendeten Beschichtungslösungen wiesen folgende Zusammensetzungen auf:

Tabelle 1: Beschichtung A: 500 mg/Liter Cremophor®EL (Caesar + Loretz GmbH, Hilden) in demineralisiertem Wasser; Beschichtung B: "Monomer PEO 550" bis zur Sättigung in demineralisiertem Wasser gelöst; Beschichtung C: "Monomer PEO 350" bis zur Sättigung in demineralisiertem Wasser gelöst; Beschichtung D: "Dimer PEO 350" bis zur Sättigung in demineralisiertem Wasser gelöst.

Tabelle 2: Beschichtung A: 100 mg/Liter Cremophor®EL (Caesar + Loretz GmbH, Hilden) in demineralisiertem Wasser; Beschichtung B: 100 mg/Liter "Dimer PEO 350" in demineralisiertem Wasser; Beschichtung C: 50 mg/Liter "Dimer PEO 350" in demineralisiertem Wasser.

### Prüfung der Hämatokompatibilität der Beschichtungen

Die so behandelten Loops mit einem Volumen von 36ml und einer inneren Oberfläche von 150 cm² wurden ebenso wie ein unbeschichteter Kontroll-Loop jeweils mit 20 ml frischem humanen Blut gefüllt und im Wasserbad rotiert.

Hierbei ist zu beachten, dass Blut unterschiedlicher Donoren verschieden auf den Kontakt mit potentiell hämoinkompatiblen Oberflächen und die während des Tests auftretende Agitation reagiert. Somit wurde, um eine größtmögliche Signifikanz der gemessenen Werte zu erzielen, in den Parallelversuchen mit verschiedenen Beschichtungen und Kontrollen stets vom gleichen Donor stammendes Blut verwendet und die gesamten Versuche mit dem Blut von fünf verschiedenen Donoren durchgeführt.

Nach 90 Minuten wurde den Loops Blut entnommen und diese Proben auf bestimmte Werte analysiert.

In Tabelle 1 sind die Anzahl der intakten Blutplättchen nach Versuchsdurchführung, sowie die Konzentrationen von β-Thromboglobulin, Thrombin-Antithrombin, Komplement SC5b-9 und PMN-Elastase für jeden getesteten Schlauch aufgeführt. Hierbei sind die aus den Versuchen mit dem Blut von fünf unterschiedlichen Donoren ermittelten Mittelwerte, sowie jeweils die Standardabweichung (SD) und die prozentuale Abweichung des Mittelwerts vom Mittelwert aus dem unbehandelten Kontrollblut aufgeführt.

Die in den Proben nachgewiesene Zahl der intakten Blutplättchen und Konzentration ausgeschütteten β-Thromboglobulins (iu/ml) sind hierbei die wichtigsten Parameter. Hierbei soll der erste Wert möglichst hoch, der zweite hingegen möglichst niedrig sein.

Der Tabelle 1 ist zu entnehmen, dass die Anzahl der intakten Blutplättchen bei der "Dimer PEO350"-Beschichtun (100 mg/l) deutlich höher ist als bei der Cremophor®EL-Beschichtung. Die beiden weiterhin getesteten Substanzen weisen eine im Vergleich zu Cremophor leicht erhöhte ("Monomer PEO550") oder nahezu gleiche ("Monomer PEO350") Blutplättchenzahl auf.

Weiterhin ist die im Test mit "Dimer PEO 350"-Beschichtung festgestellte β-Thromboglobulin-Konzentration gegenüber der Cremophor®EL-Beschichtung um fast ein Drittel verringert. Dieser Unterschied lässt auf eine wesentlich bessere Hämatokompatibilität der "Dimer PEO 350"-Beschichtung gegenüber der Cremophor®EL-Beschichtung schließen. Auch die beiden monomeren Substanzen weisen hier im Vergleich zu Cremophor®EL deutlich bessere Werte auf.

Der weiterhin getestete Thrombin-Antithrombin Wert zeigt, dass auch hier die "Dimer PEO 350"-Beschichtung der Cremophor®EL-Beschichtung hinsichtlich ihrer Hämatokompatibilität überlegen ist.

**Tabelle 1: Vergleich von "Monomer PEO 550", "Monomer PEO 350" und "Dimer "PEO 350" mit Cremophor®EL /500 mg**

| | Kontrollblut (frisches Donorblut) | | | Unbeschichteter Loop (PC+Silicon) | | | Cremophor®EL (500 mg/l) | | | "Monomer PEO 550" gesättigt | | | "Monomer PEO 350" gesättigt | | | "Dimer PEO 350" gesättigt | | |
|---|---|---|---|---|---|---|---|---|---|---|---|---|---|---|---|---|---|---|
| Plateletzahl (x10³/µl) (SD) | 219 | | (32) | 110 | | (28) | 109 | | (41) | 119 | | (49) | 107 | | (50) | 143 | | (61) |
| % | 100 | | | 50 | | | 50 | | | 54 | | | 49 | | | 65 | | |
| β-Thrombolglobin (iu/ml) (SD) | 225 | | (269) | 2948 | | (1691) | 3567 | | (2614) | 2627 | | (1621) | 2283 | | (1602) | 1610 | | (1238) |
| % | 100 | | | 1308 | | | 1582 | | | 1165 | | | 1013 | | | 714 | | |
| Thrombin-Antithrombin (µg/l)(SD) | 23 | | (27) | 38 | | (19) | 29 | | (10) | 46 | | (21) | 36 | | (19) | 31 | | (18) |
| % | 100 | | | 163 | | | 124 | | | 197 | | | 153 | | | 134 | | |
| Complement SC5b-9 (µg/l) (SD) | 155 | | (44) | 877 | | (226) | 1674 | | (812) | 1948 | | (920) | 1796 | | (809) | 1911 | | (795) |
| % | 100 | | | 566 | | | 1080 | | | 1260 | | | 1159 | | | 1233 | | |
| PMN-Elastase (µg/l) (SD) | 30 | | (5) | 63 | | (15) | 62 | | (16) | 68 | | (17) | 75 | | (29) | 61 | | (15) |
| % | 100 | | | 210 | | | 206 | | | 226 | | | 249 | | | 204 | | |

| | | | | | | | | | | | | | | | | | | |
|---|---|---|---|---|---|---|---|---|---|---|---|---|---|---|---|---|---|---|
| (iu: international unit; SD: Standardabweichung) | | | | | | | | | | | | | | | | | | |

Aus den weiteren, in Tabelle 1 aufgeführten Ergebnissen für Komplement SC5b-9 und PMN-Elastase, wobei jeweils niedrigere Werte eine bessere Hämatokompatibilität anzeigen, wird ersichtlich, dass die drei getesteten neuen Substanzen hinsichtlich dieser Parameter Cremophor®EL ebenbürtig bzw. nur geringfügig abweichend wirken. Hierbei sollte erwähnt werden, dass das "Dimer PEO 350" mit Ausnahme des Komplement-SC5b-9-Wertes durchgehend bessere Werte zeigt als die getesteten Monomere.

Darüber hinaus zeigen die in Tabelle 2 aufgeführten Ergebnisse, dass sich die Hämatokompatibilität der "Dimer PEO 350"-Beschichtung durch eine Verringerung der eingesetzten "Dimer PEO 350"-Konzentration um die Hälfte (50mg/l) nochmals deutlich steigern lässt.In Tabelle 2 sind die Ergebnisse eines Tests aufgeführt, in dem analog zu dem in Tabelle 1 dargestellten Test die Wirksamkeit von Beschichtungslösungen mit verschiedenen definierten Konzentrationen von "Dimer PEO 350" im Vergleich zur Wirksamkeit einer Cremophor®EL enthaltenden Beschichtungslösung getestet wurde.

Hierbei zeigt sich, dass die Beschichtung mit "Dimer PEO350" bei gleicher Konzentration (mg/l) der Beschichtungslösung beinahe durchgehend eine bessere Hämatokompatibilität aufweist als die Beschichtung mit Cremophor®EL.

Diese Tendenz lässt sich durch eine weitere Verringerung der "Dimer PEO 350"-Konzentration der Beschichtungslösung (mg/l) sogar noch verstärken.

Die Ergebnisse des Chandler-Loop-Tests zeigen somit, dass die neuen Albumin-Analoga mit einer räumlich definierten Grundstruktur gegenüber den bekannten Substanzen auf Basis nichtionischer Ester mit einer räumlich undefinierten Grundstruktur verbesserte Eigenschaften bezüglich Hämatokompatibilität und Antithrombogenizität aufweisen.

**Tabelle 2:Vergleich von "Dimer PEO 350" in niedriger Konzentration mit Cremophor®EL (100 mg/l)**

| | Kontrollblut (frisches Donorblut) | | | Unbeschichteter Loop (PC + Silicon) | | | Cremophor®EL (100 mg/l) | | | "Dimer PEO 350" (100 mg/l | | | Dimer PEO 350" (50 mg/l) | | |
|---|---|---|---|---|---|---|---|---|---|---|---|---|---|---|---|
| Plateletzahl (x10³/µl) (SD) | 242 | | (34) | 143 | | (21) | 160 | | (44) | 183 | | (43) | 184 | | (36) |
| % | 100 | | | 59 | | | 66 | | | 76 | | | 72 | | |
| β-Thromboglobulin (iu/ml) (SD) | 120 | | (24) | 1878 | | (787) | 2501 | | (868) | 1725 | | (587) | 1504 | | (552) |
| % | 100 | | | 1560 | | | 2077 | | | 1432 | | | 1249 | | |
| Thrombin-Antithrombin (µg/l)(SD) | 2,5 | | (0,1) | 14,9 | | (19) | 28,8 | | (23,7) | 18,9 | | (10,0) | 9,6 | | (3,6) |
| % | 100 | | | 588 | | | 1139 | | | 746 | | | 381 | | |
| Complement SC5b-9 (µg/l) (SD) | 189 | | (24) | 996 | | (340) | 2481 | | (818) | 2672 | | (722) | 2481 | | (715) |
| | 100 | | | 527 | | | 1312 | | | 1413 | | | 1312 | | |
| PMN-Elastase (µg/l) (SD) | 36 | | (5) | 71 | | (8) | 79 | | (12) | 77 | | (12) | 74 | | (9) |
| % | 100 | | | 200 | | | 222 | | | 217 | | | 207 | | |

| | | | | | | | | | | | | | | | |
|---|---|---|---|---|---|---|---|---|---|---|---|---|---|---|---|
| (iu: international unit; SD: Standardabweichung) | | | | | | | | | | | | | | | |

## Patentansprüche

1. Vollsynthetisches Albumin-Analogon, **dadurch gekennzeichnet, dass** es zwei über eine Verbrückung (20) miteinander verbundene dreidimensionale Grundstrukturen (11), mit jeweils mindestens zwei gebundenen Gelenkbereichen (12) in geometrisch zueinander definierter Weise aufweist, an denen jeweils ein Rest (14) kovalent gebunden ist, wobei die Grundstrukturen (11) jeweils eine Benzolcarbonsäure sind, und wobei die Gelenkbereiche (12) durch Säureamidbindungen gebildet sind, und wobei der Rest (14) jeweils einen lipophilen Bereich und einen hydrophilen Bereich aufweist.

2. Vollsynthetisches Albumin-Analogon nach Anspruch 1, **dadurch gekennzeichnet, dass** ein Kettenabschluss (17) an den Resten (14) mit organischen Resten abgesättigt ist.

3. Vollsynthetisches Albumin-Analogon nach einem der Ansprüche 1 oder 2, **dadurch gekennzeichnet, dass** Reste (14) miteinander durch mindestens eine Brücke (19) verbunden sind.

4. Vollsynthetisches Albumin-Analogon nach einem der Ansprüche 1 bis 3, **dadurch gekennzeichnet, dass** die Verbrückung (20) eine Alkylbrücke der Formel (CH₂)ₙ mit einer Länge von n = 2 bis 18 aufweist.

5. Vollsynthetisches Albumin-Analogon nach einem der Ansprüche 1 bis 4, **dadurch gekennzeichnet, dass** die Verbrückung (20) mindestens eine Verzweigung (21) aufweist, an der ein Rest (14) gebunden ist.

6. Vollsynthetisches Albumin-Analogon nach einem der Ansprüche 1 bis 5, **dadurch gekennzeichnet, dass** an einigen Gelenkbereichen (12), Resten (14) und/oder Verbrückungen (20) hydrophile Funktionen (22), bspw. mit mehreren ionischen Gruppen wie etwa Carboxylat-, Phosphat- oder Sulfonat-Anionen, eingeführt sind.

7. Vollsynthetisches Albumin-Analogon nach einem der Ansprüche 1 bis 6, **dadurch gekennzeichnet, dass** der lipophile Bereich des Restes durch zumindest eine Alkylkette gebildet wird.

8. Vollsynthetisches Albumin-Analogon nach einem der Ansprüche 1 bis 7, **dadurch gekennzeichnet, dass** der hydrophile Bereich des Restes durch zumindest eine Polyethylen-Oxid-Kette gebildet wird.

9. Vollsynthetisches Albumin-Analogon nach einem der Ansprüche 1 bis 8, **dadurch gekennzeichnet, dass** es mindestens 4 über die Gelenkbereiche gebundene Reste aufweist.

10. Vollsynthetisches Albumin-Analogon nach einem der Ansprüche 1 bis 9, **dadurch gekennzeichnet, dass** es die Struktur wie in Fig. 5 dargestellt aufweist, in welcher R = 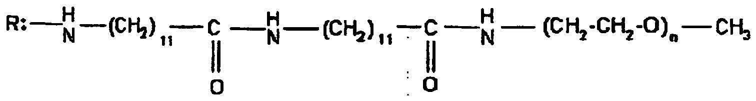 wobei n eine ganze Zahl von 8 bis 13 ist.

11. Hämatokompatible Beschichtung für mit Blut in Kontakt kommende Oberflächen, die ein vollsynthetisches Albumin-Analogon nach einem der Ansprüche 1 bis 10 aufweist.

12. Medizinische Vorrichtung mit zumindest einer Oberfläche, die eine hämokompatible Beschichtung nach Anspruch 11 aufweist.

13. Medizinische Vorrichtung nach Anspruch 12, **dadurch gekennzeichnet, dass** sie Bestandteil von Einmalgeräten einer Herz-Lungen-Maschine, eines Oxygenators, eines Katheters, eines künstlichen Herzens, einer künstlichen Niere, einer Gasaustauschmembran oder einer vaskulären Prothese ist.

14. Medizinische Vorrichtung nach einem der Ansprüche 12 oder 13, **dadurch gekennzeichnet, dass** die Oberfläche Polycarbonat, Polyethylen, Polypropylen, Polymethylpenten, Polyurethan, Polyester, Silikon, Hart- oder Weich-Polyvinylchlorid, Copolymer, Acrylnitril-Butadien-Styrol-Copolymer und/oder Ethylen-Propylen-(Dien)-Copolymer enthält.

15. Verwendung eines vollsynthetischen Albumin-Analogons nach einem der Ansprüche 1 bis 10 in einer Beschichtung für mit Blut in Kontakt kommende Oberflächen.

## Claims

1. A fully synthetic albumin analog **characterized in that** it has two three-dimensional basic structures (11) which are connected with one another via one bridging unit (20), and which have in a geometrically defined manner, at least two bound joint regions (12) to which at least one residue (14) is covalently bound, wherein each of the basic structures (11) is a benzoic acid, and wherein the joint regions (12) are formed by acid amide bonds, and wherein the residue (14) comprises, respectively, a lipophilic region and a hydrophilic region.

2. The fully synthetic albumin analog as claimed in claim 1, **characterized in that** a chain terminal (17) on residues (14) is saturated with organic radicals.

3. The fully synthetic albumin analog as claimed in any of claims 1 or 2, **characterized in that** residues (14) are connected with one another by at least one bridge (19).

4. The fully synthetic albumin analog as claimed in any of claims 1 to 3, **characterized in that** the bridging unit (20) has an alkyl bridge of the formula (CH₂)ₙ having a length of n = 2 to 18.

5. The fully synthetic albumin analog as claimed in any of claims 1 to 4, **characterized in that** the bridging unit (20) has at least one branch (21) to which a residue (14) is bound.

6. The fully synthetic albumin analog as claimed in any of claims 1 to 5, **characterized in that** hydrophilic functions (22), having for example, multiple ionic groups such as carboxylate, phosphate, or sulfonate anions, have been introduced at some joint regions (12), residues (14), and/or bridging units (20).

7. The fully synthetic albumin analog as claimed in any of claims 1 to 6, **characterized in that** the lipophilic region of the residue is formed by at least one alkyl chain.

8. The fully synthetic albumin analog as claimed in any of claims 1 to 7, **characterized in that** the hydrophilic region of the residue is formed by at least one polyethylene oxide chain.

9. The fully synthetic albumin analog as claimed in any of claims 1 to 8, **characterized in that** it comprises at least 4 residues bound via the joint regions.

10. The fully synthetic albumin analog as claimed in any of claims 1 to 9, **characterized in that** it has the structure as shown in Fig. 5, wherein R = 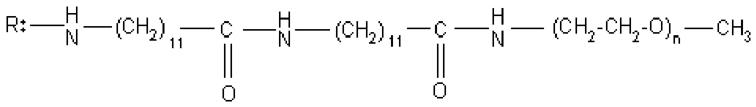 wherein n is an integral number of from 8 to 13.

11. A hemocompatible coating for surfaces which come into contact with blood, said coating having at least one fully synthetic albumin analog as claimed in any of claims 1 to 10.

12. A medical device having at least one surface which has a coating as claimed in claim 11.

13. The medical device as claimed in claim 12, **characterized in that** it is a component of diposable equipment of a heart-lung machine, of an oxygenator, of a catheter, of an artificial heart, of an artificial kidney, of a gas exchange membrane, or of a vascular prosthesis.

14. The medical device as claimed in either of claims 12 or 13, **characterized in that** the plastics surface comprises polycarbonate, polyethylene, polypropylene, polymethylpentene, polyurethane, polyester, silicone, hard or soft polyvinyl chloride, copolymers, acrylonitrile butadiene styrene copolymer, and/or ethylene propylene (diene) copolymer.

15. The use of a fully synthetic albumin analog as claimed in any of claims 1 to 10 in a coating for polymer surfaces which come into contact with blood.

## Revendications

1. Analogue entièrement synthétique de l'albumine, **caractérisé en ce qu'**il comprend deux structures de base tridimensionnelles (11), reliées l'une à l'autre par un pontage (20), ayant chacune au moins deux domaines charnières liés (12) d'une manière géométriquement définie l'un par rapport à l'autre, à chacun desquels un résidu (14) est lié par une liaison covalente, chacune des structures de base (11) étant un acide benzènecarboxylique, et les domaines charnières (12) étant formés par des liaisons amide d'acide, et chaque résidu (14) comportant un domaine lipophile et un domaine hydrophile.

2. Analogue entièrement synthétique de l'albumine selon la revendication 1, **caractérisé en ce qu'**un terminateur de chaîne (17), sur les résidus (14), est saturé par des résidus organiques.

3. Analogue entièrement synthétique de l'albumine selon l'une des revendications 1 ou 2, **caractérisé en ce que** les résidus (14) sont reliés l'un à l'autre par au moins un pont (19).

4. Analogue entièrement synthétique de l'albumine selon l'une des revendications 1 à 3, **caractérisé en ce que** le pontage (20) comprend un pont alkyle de formule (CH₂)ₙ, ayant une longueur n = 2 à 18.

5. Analogue entièrement synthétique de l'albumine selon l'une des revendications 1 à 4, **caractérisé en ce que** le pontage (20) comprend au moins une ramification (21) à laquelle est lié un résidu (14).

6. Analogue entièrement synthétique de l'albumine selon l'une des revendications 1 à 5, **caractérisé en ce que** des fonctions hydrophiles (22), ayant par exemple plusieurs groupes ioniques, tels que des anions carboxylate, phosphate ou sulfonate, sont insérés dans quelques domaines charnières (12), résidus (14) et/ou pontages (20).

7. Analogue entièrement synthétique de l'albumine selon l'une des revendications 1 à 6, **caractérisé en ce que** le domaine lipophile du résidu est formé d'au moins une chaîne alkyle.

8. Analogue entièrement synthétique de l'albumine selon l'une des revendications 1 à 7, **caractérisé en ce que** le domaine hydrophile du résidu est formé d'au moins une chaîne poly(oxyde d'éthylène).

9. Analogue entièrement synthétique de l'albumine selon l'une des revendications 1 à 8, **caractérisé en ce qu'**il comprend au moins 4 résidus liés par l'intermédiaire des domaines charnières.

10. Analogue entièrement synthétique de l'albumine selon l'une des revendications 1 à 9, **caractérisé en ce qu'**il a la structure telle que représentée sur la Figure 5, dans laquelle R est 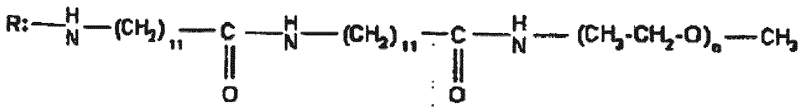 où n est un nombre entier de 8 à 13.

11. Revêtement hématocompatible pour surfaces entrant en contact avec le sang, qui comprend un analogue entièrement synthétique de l'albumine selon l'une des revendications 1 à 10.

12. Dispositif médical ayant au moins une surface qui présente un revêtement hématocompatible selon la revendication 11.

13. Dispositif médical selon la revendication 12, **caractérisé en ce qu'**il est un constituant d'appareils à usage unique d'un coeur-poumon artificiel, d'un oxygénateur, d'un cathéter, d'un coeur artificiel, d'un rein artificiel, d'une membrane d'échange gazeux ou d'une prothèse vasculaire.

14. Dispositif médical selon l'une des revendications 12 ou 13, **caractérisé en ce que** la surface contient du polycarbonate, du polyéthylène, du polypropylène, du polyméthylpentène, du polyuréthanne, un polyester, de la silicone, du poly(chlorure de vinyle) rigide ou souple, un copolymère, un copolymère acrylonitrile-butadiène-styrène et/ou un copolymère éthylène-propylène-(diène).

15. Utilisation d'un analogue entièrement synthétique de l'albumine selon l'une des revendications 1 à 10 dans un revêtement pour surfaces entrant en contact avec le sang.
